# EUROPÄISCHE PATENTSCHRIFT

(11) **EP 2 619 415 B1**
(45) Veröffentlichungstag und Bekanntmachung des Hinweises auf die Patenterteilung: **08.04.2015**
(21) Anmeldenummer: 11775727.8
(22) Anmeldetag: 10.08.2011
(51) Int. Cl.: F01B 17/02, F01B 11/00, A61M 1/00

(54) **DRUCKGASMOTOR FÜR EIN LAVAGESYSTEM**
COMPRESSED GAS MOTOR FOR A WASH SYSTEM
MOTEUR À GAZ COMPRIMÉ POUR UN SYSTÈME DE LAVAGE

(30) Priorität: 22.09.2010 DE 102010046057
(43) Veröffentlichungstag der Anmeldung: 31.07.2013
(73) Patentinhaber: Heraeus Medical GmbH, 61273 Wehrheim (DE)
(72) Erfinder: VOGT, Sebastian, 99092 Erfurt (DE); BUECHNER, Hubert, 90491 Nuernberg (DE)
(74) Vertreter: Schultheiss & Sterzel Patentanwälte PartG mbB
(86) Internationale Anmeldenummer: PCT/EP2011/003993
(87) Internationale Veröffentlichungsnummer: WO 2012/038003

(56) Entgegenhaltungen:
- WO-A1-96/00524

## Beschreibung

Die Erfindung betrifft einen Druckgasmotor zum Betreiben eines Lavagesystems umfassend einen zylindrischen Innenraum, der an zumindest einer ersten Grundfläche gasdicht verschlossen ist, zumindest einen Kolben, der beweglich entlang der Zylinderachse im zylindrischen Innenraum angeordnet ist und der dicht mit dem Innenraum schließt, eine Kolbenstange, die mit dem Kolben verbunden ist und die über die zweite Grundfläche aus dem Innenraum hinaus ragt, eine erste Öffnung zum Einspeisen eines komprimierten Gases in den Innenraum und eine zweite Öffnung zum Ableiten eines Gases aus dem Inneren des Innenraums.

Die Erfindung betrifft auch ein Handgriff für ein Lavagesystem mit einem solchen Druckgasmotor sowie ein Lavagesystem mit einem solchen Druckgasmotor und ein Verfahren zum Betreiben eines solchen Druckgasmotors.

Gegenstand der Erfindung ist also eine einfache Antriebsvorrichtung zur Erzeugung von oszillierenden, linearen Bewegungen unter Verwendung von komprimiertem Gas als Antriebsmittel für ein Lavagesystem. Weiterhin ist ein Handgriff für ein Lavagesystem mit der Antriebsvorrichtung Gegenstand der Erfindung. Des Weiteren ist auch ein Lavagesystem mit der Antriebsvorrichtung und einem Handgriff Gegenstand der Erfindung.

Lavagesysteme werden in der Chirurgie in breitem Umfang eingesetzt, um Gewebeareale zu reinigen. Insbesondere bei der Implantation von Gelenkendoprothesen und bei septischen Revisionen sind Lavagesysteme von wesentlicher Bedeutung (R. M. Sherman et al.: The role of lavage in preventing hemodynamic and blood-gas changes during demented arthroplasty. J. Bone Joint. Surg. 1983; 65-A: 500-506.; S. J. Breusch et al.: Zementierte Hüftendoprothetik: Verminderung des Fettembolierisikos in der zementierten Hüftendoprothetik mittels gepulster Druckspülung. Orthopädie 2000; 29: 578-586.; S. J. Breusch et al.: Lavage technique in THA: Jet-lavage Produces Better Cement Penetration Than Syringe-Lavage in the Proximal Femur. J. Arthroplasty. 200; 15(7): 921-927.; R. J. Byrick et al.: High-volume, high pressure pulsatile lavage during cemented arthroplasty. J. Bone Joint Surg. 1989; 81-A: 1331-1336.; J. Christie et al.: Medullary lavage reduces embolic phenomena and cardiopulmonary changes during cemented hemiarthorplasty. J. Bone Joint Surg. 1995; 77-B: 456-459.) Die Reinigung der Gewebeareale erfolgt bei der Lavage durch Sprühstöße mit geeigneten Spülflüssigkeiten, wie isotonischen Kochsalzlösungen. Üblich sind dabei bis zu mehreren tausend Sprühstößen pro Minute. Gepulste Lavagesysteme sind seit langem bekannt, beispielsweise aus der US 4,583,531 A, der US 4,278,078 A und der US 5,542,918 A. Die gegenwärtig im Markt befindlichen Lavagesysteme werden durch Elektromotoren (zum Beispiel InterPulse® Jet lavage der Stryker GmbH & Co. KG) oder durch Druckluft (zum Beispiel PALAVAGE® der Heraeus Medical GmbH) angetrieben.

Bewährt haben sich beispielsweise batteriebetriebene Lavagesysteme. Es muss jedoch immer ein großer Batterieblock mitgeführt werden, der naturgemäß nur eine begrenzte Ladungskapazität hat. Druckluftgetriebene Lavage-Systeme haben dagegen den Vorteil, dass Druckluft im Operationssaal unlimitiert zur Verfügung steht und damit beliebig lange Spülflüssigkeit versprüht werden kann, ohne dass die Energiezufuhr begrenzt ist.

Bei den mit Druckluft oder einem anderen komprimierten Gas getriebenen Systemen wird üblicherweise ein Druckgasmotor zum Antrieb eingesetzt. Bei den meisten Druckgasmotoren für Lavagesysteme handelt es sich um Lamellen-Druckgasmotoren. Der Druckgasmotor erzeugt eine Drehbewegung, die dann in eine oszillierende, lineare Bewegung umgesetzt wird. Die oszillierende, lineare Bewegung wird genutzt, um jeweils kleinen Volumina des Spülmediums einen Impuls zu verleihen. Dabei wird üblicherweise mindestens eine Membran zwischen dem Antrieb und dem Zulauf der Spülflüssigkeit angeordnet, um die Impulse auf die Spülflüssigkeit übertragen zu können. Es entstehen dadurch Sprühstöße. Bei hohen Pulszahlen von 2000 bis 3000 Impulsen pro Minute werden dabei Volumina im Bereich von mehreren hundert Millilitern Spülflüssigkeit versprüht. Das bedeutet, dass der Druckgasmotor sehr präzise gefertigt sein muss, um entsprechend hohe Drehzahlen zu tolerieren. Weiterhin muss eine entsprechend stabile Lagerung vorhanden sein. Aus diesen Gründen ist der Druckgasmotor bei üblichen Druckluft-getriebenen Lavage-Systemen das kostenintensivste Bauteil. Es wird deshalb im Allgemeinen der Druckgasmotor in einem Handgriff aus Metall oder anderen langzeitstabilen Materialien angeordnet, so dass dieses Bauteil mehrfach nach entsprechender Aufbereitung und Sterilisation genutzt werden kann.

Die WO 96/00524 A1 offenbart ein Lavage-System mit einem Druckgasmotor, bei dem ein ausströmendes Gas oder ein Teil davon zur Beschleunigung eines Flüssigkeitsstrahls für medizinische Spülsysteme verwendet wird. Aus der EP 0 481 208 A1 ist ein gattungsgemäßer Druckgasmotor mit pneumatisch betriebenen Umsteuerventilen bekannt, die seitlich neben einem Kolben angeordnet sind, der linear in einem zylindrischen Hohlraum beweglich ist. Bei diesem Aufbau steuern die Umsteuerventile die den Kolben antreibende Antriebsdruckluft. Der Kolben wird abwechselnd von beiden Seiten mit Druckluft beaufschlagt, um eine lineare, oszillierende Bewegung des Kolbens zu verursachen.
Nachteilig ist hieran, dass der Aufbau der Umsteuerventile relativ kompliziert und dadurch auch aufwendig im Aufbau ist. Ein solcher Aufbau kann daher nicht besonders kostengünstig realisiert werden.

Ein Beispiel für eine Druckluft getriebene Pumpe ist in EP 0 378 434 A2 offenbart. Die Pumpe enthält einen Kolben und einen Stopfen, die beide unter Federspannung stehen. Durch Beaufschlagen mit Druckluft entsteht durch ein Zusammenspiel von Kolben, Stopfen und Federn eine oszillierende Bewegung, die über Ventile auf einen Flüssigkeitsstrom übertragen wird, so dass ein gepulster Strahl erzeugt wird.

In JP 2002061566 A ist ferner eine Pumpe offenbart, die aus zwei zusammengesetzten Blöcken besteht, von denen einer einen Kolben und der andere ein Schaltventil enthält. Zwischen beiden Komponenten wird eine Leitungsplatte gesetzt, die Öffnungen zum Kolben und vom Schaltventil auf geeignete Weise miteinander verbindet.

Der Erfindung liegt die Aufgabe zugrunde, die Nachteile des Stands der Technik zu überwinden, insbesondere soll ein einfacher Druckgasmotor für ein Lavagesystem bereitgestellt werden, der aus möglichst wenigen Teilen besteht und durch komprimiertes Gas antreibbar ist. Diese Antriebsvorrichtung sollte dabei weitgehend aus kostengünstigen Bauteilen bestehen. Dadurch soll ermöglicht werden, eine Antriebsvorrichtung für den einmaligen Gebrauch mit geringen Herstellungs- und Montagekosten bereitzustellen. Es sollen kostenintensive Teile, die eine präzise, stabile Lagerung erfordern, möglichst vermieden werden. Die Aufgabe betrifft wieterhin einen Handgriff für ein Lavagesystem sowie ein Lavagesystem mit oben angeführtem Druckgasmotor sowie ein Verfahren zum Betreiben eines solchen Druckgasmotors.

Die Aufgabe der Erfindung wird durch die Merkmale des Anspruchs 1, sowie der unabhängigen Ansprüche 21, 22 und 23 gelöst, insbesondere durch einen Druckgasmotor für ein Lavagesystem, dessen Öffnungen im Zylindermantel des zylindrischen Innenraums angeordnet sind, wobei die erste Öffnung zwischen der zweiten Öffnung und der verschlossenen ersten Grundfläche angeordnet ist, der Kolben ein erstes Kolbenteil und ein zweites Kolbenteil umfasst, die miteinander verbunden sind, wobei das erste Kolbenteil zumindest einen Durchgang umfasst und/oder zwischen dem ersten Kolbenteil und der Zylinderwand des Innenraums zumindest einen Durchgang bildet und das zweite Kolbenteil das Innere des Innenraums dicht schließt, wobei zumindest ein Durchgang des ersten Kolbenteils einen Zwischenraum zwischen dem ersten und dem zweiten Kolbenteil mit einem Innenraumbereich zwischen der verschlossenen ersten Grundfläche und dem ersten Kolbenteil verbindet und wobei die erste Öffnung durch das erste Kolbenteil und die zweite Öffnung durch das zweite Kolbenteil verschließbar sind und der Druckgasmotor eine Rückstelleinrichtung umfasst, die zumindest zeitweise eine Kraft in Richtung der ersten Grundfläche des zylindrischen Innenraums auf den Kolben ausübt, wenn das zweite Kolbenteil die zweite Öffnung nicht vollständig verschließt.

Ein Hohlzylinder im Sinne der vorliegenden Erfindung bezieht sich auf den inneren Hohlzylinder. Wie bei einem Motor im Allgemeinen üblich, benötigt das Teil, zum Beispiel der Motorblock, in dem der Zylinder ausgebildet ist, keine zylindrische Symmetrie. Unter einem Hohlzylinder im Sinne der vorliegenden Erfindung ist des Weiteren nicht nur ein Körper mit einfacher zylindrischer Geometrie zu verstehen, sondern der Begriff umfasst auch Hohlzylinder mit nichtrunder, beispielsweise rechteckiger oder sogar unregelmäßig geformter Grundfläche. Der Hohlzylinder muss in dem Bereich, in dem sich die Kolben bewegen, eine gleichbleibende Querschnittsform senkrecht zur Zylinderachse aufweisen, also einen Zylinder in der allgemeinsten Form darstellen.

Es ist erfindungsgemäß, dass der Zwischenraum ein konstantes Volumen aufweist, während das Volumen des Innenraumbereichs von der Position der Kolbenteile relativ zum Innenraum abhängt.

Es kann auch vorgesehen sein, dass die Wand des Innenraums eine Furche umfasst, die sich von dem Bereich des Abschlusses der ersten Grundfläche des Innenraums bis zum Bereich der Höhe der zweiten Öffnung erstreckt und die zumindest einen Durchgang zwischen dem ersten Kolbenteil und der Zylinderwand des Innenraums bildet, wobei die Furche nicht mit den Öffnungen verbunden ist.

Dabei kann wiederum vorgesehen sein, dass die Furche sich bis in den Abschluss der ersten Grundfläche des zylindrischen Innenraums erstreckt und das vorzugsweise das erste Kolbenteil ein zylindrischer Körper ist, der besonders bevorzugt keine weiteren Durchgänge umfasst.

Erfindungsgemäße Druckgasmotoren können sich auch dadurch auszeichnen, dass das erste Kolbenteil und das zweite Kolbenteil über eine starre Verbindung, bevorzugt über eine Stange, besonders bevorzugt über eine Verlängerung der Kolbenstange, insbesondere entlang der Zylinderachse miteinander verbunden sind.

Auch kann vorgesehen sein, dass die zweite Grundfläche des zylindrischen Innenraums offen ist.

Es kann ferner erfindungsgemäß vorgesehen sein, dass an der zweiten Grundfläche des zylindrischen Innenraums eine Halterung für die Kolbenstange angeordnet ist, die die Kolbenstange vorzugsweise zentrisch im Bereich der Zylinderachse des Innenraums lagert.

Es kann auch vorgesehen sein, dass der zumindest eine Durchgang zumindest eine durchgehende, gasdurchlässige Bohrung im ersten Kolbenteil umfasst, insbesondere eine solche Bohrung ist.

Eine weitere Ausgestaltung der Erfindung sieht vor, dass der Abstand zwischen dem ersten Kolbenteil und dem zweiten Kolbenteil größer als 1 mm, bevorzugt zwischen 1 mm und 100 mm und besonders bevorzugt zwischen 5 mm und 10 mm ist.

Auch kann vorgesehen sein, dass der Abstand der ersten Öffnung zur verschlossenen ersten Grundfläche mindestens so groß ist wie die Höhe des ersten Kolbenteils in Richtung der Zylinderachse des Innenraums.

Erfindungsgemäße Druckgasmotoren können sich auch dadurch auszeichnen, dass das erste Kolbenteil und/oder das zweite Kolbenteil zumindest bereichsweise zylindrisch sind und dicht an der Innenwand des zylindrischen Innenraums anliegen.

Es kann vorgesehen sein, dass an der ersten Öffnung ein Reservoir angeschlossen ist, das ein komprimiertes Gas beinhaltet und/oder die zweite Öffnung gasdurchlässig mit der Umgebung des Druckgasmotors verbunden ist, vorzugsweise ohne den Gasstrom behindernde Leitungen. Ferner kann vorgesehen sein, dass außen im Bereich der zweiten Grundfläche ein Befestigungsmittel zum Befestigen eines Lavagevorsatzes, insbesondere ein Bajonettverschluss angeordnet ist.

Es kann vorteilhaft sein, dass das aus der zweiten Grundfläche herausragenden Ende der Kolbenstange einen Stößel, insbesondere einen pilzförmiger Stößel, umfasst.

Besonders kostengünstig sind erfindungsgemäße Druckgasmotoren, wenn die Kolbenteile, die Verbindungen der Kolbenteile, die Kolbenstange, die Wände des zylindrischen Innenraums, der oder die Abschlüsse der Grundflächen, die Halterung, die Rückstelleinrichtung, die Befestigungsmittel und/oder der Stößel aus Kunststoff gefertigt sind, insbesondere Spritzgießteile sind.

Auch kann vorgesehen sein, dass der Innenraum das Innere eines zylindrischen Rohrs ist und das der Druckgasmotor zumindest bereichsweise, vorzugsweise im Wesentlichen, eine zylindrische Außenfläche umfasst.

Besonders bevorzugt ist vorgesehen, dass bei einer oszillierenden Bewegung des Kolbens im Innenraum die erste Öffnung durch das erste Kolbenteil und die zweite Öffnung durch das zweite Kolbenteil abwechselnd vollständig verschließbar sind.

Erfindungsgemäß kann vorgesehen sein, dass die Rückstelleinrichtung eine Feder, insbesondere eine Plastik-, Stahlfeder und/oder Gasfeder umfasst, die zwischen dem zweiten Kolbenteil und der zweiten Grundfläche des zylindrischen Innenraums angeordnet ist.

Alternativ hierzu kann vorgesehen sein, dass die Rückstelleinrichtung einen dritten Kolbenteil, eine dritte Öffnung zum Ableiten eines Gases aus dem Inneren des Innenraums und eine vierte Öffnung zum Einspeisen eines komprimierten Gases in den Innenraum umfasst, wobei die dritte und vierte Öffnung im Zylindermantel des zylindrischen Innenraums angeordnet sind, die vierte Öffnung zwischen der zweiten Öffnung und der zweiten Grundfläche des zylindrischen Innenraums angeordnet ist, die dritte Öffnung zwischen der zweiten Öffnung und der vierten Öffnung angeordnet ist, die dritte Öffnung durch das zweite Kolbenteil und die vierte Öffnung durch das dritte Kolbenteil verschließbar sind, und wobei das dritte Kolbenteil ein Teil des Kolbens ist, das an der Kolbenstange zwischen dem zweiten Kolbenteil und der zweiten Grundfläche des zylindrischen Innenraums angeordnet ist und mit dem zweiten Kolbenteil verbunden ist, und das dritte Kolbenteil zumindest einen Durchgang umfasst und/oder zwischen dem dritten Kolbenteil und der Zylinderwand des Innenraums zumindest einen Durchgang bildet.

Dabei kann vorgesehen sein, dass das dritte Kolbenteil wie ein solches erstes Kolbenteil aufgebaut ist, und/oder die Innenwand des Innenraums in Bezug auf das dritte Kolbenteil so aufgebaut ist wie in Bezug auf das erste Kolbenteil.

Für erfindungsgemäße Druckgasmotoren kann auch vorgesehen sein, dass die Kolbenstange starr und unbeweglich mit dem Kolben verbunden ist, vorzugsweise mit dem zweiten Kolbenteil oder dem dritten Kolbenteil verbunden ist, insbesondere im Bereich der Zylinderachse.

Die Aufgabe der Erfindung wird auch gelöst durch einen Handgriff für ein Lavagesystem umfassend einen solchen Druckgasmotor, umfassend ein Ventil und eine am Handgriff angeordnete Bedieneinrichtung zur Steuerung des Ventils, wobei das Ventil in einer Leitung, insbesondere einer Druckgasleitung, angeordnet ist, die mit der ersten Öffnung und/oder zweiten Öffnung, vorzugsweise mit der ersten und besonders bevorzugt mit der ersten und vierten Öffnung, verbunden ist.

Die Aufgabe der Erfindung wird ferner gelöst durch ein Lavagesystem umfassend einen solchen Druckgasmotor, bevorzugt umfassend einen solchen Handgriff, des Weiteren umfassend ein Spülflüssigkeitsreservoir und/oder einen Anschluss für ein Spülflüssigkeitsreservoir, wobei über das aus der zweiten Grundfläche herausragende Ende der Kolbenstange, insbesondere über den Stößel, Kraftstöße auf eine Kraftübertragungsvorrichtung, vorzugsweise eine Membran, übertragbar sind, um Sprühstöße mit der Spülflüssigkeit zu erzeugen.

Schließlich wird die Aufgabe gelöst durch ein Verfahren zum Betreiben eines solchen Druckgasmotors, umfassend die folgenden Schritte:
Einspeisen eines komprimierten Gases durch die erste Öffnung in einen Zwischenraum zwischen dem ersten und dem zweiten Kolbenteil,
Aufbauen eines Überdrucks in dem Zwischenraum,
Bewegung der Kolbenteile im Innenraum des Druckgasmotors aufgrund des Überdrucks, Schließen der ersten Öffnung und Öffnen der zweiten Öffnung durch die Bewegung der Kolbenteile,
Ablassen des expandierten Gases durch die zweite Öffnung aus dem Zwischenraum, Rückstellen der Kolbenteile im Innenraum durch eine Rückstelleinrichtung.

Dichtungsmittel, wie O-Ringe, können bei einem erfindungsgemäßen Aufbau ebenfalls weitgehend vermieden werden.

Der Erfindung liegt also die überraschende Erkenntnis zu Grunde, dass durch einfache Mittel ein linear oszillierender Druckluftkolbenmotor beziehungsweise ein linearer Druckgaskolbenmotor aufgebaut werden kann, indem ein Kolben aus zumindest zwei Kolbenteilen und einer möglichst starren Verbindung aufgebaut wird. Eine nicht starre Verbindung verschlechtert die Leistung des Motors, ist aber der Funktionsweise nicht grundsätzlich abträglich, zudem können sich daraus positive Eigenschaften für den stabilen Lauf des Druckgasmotors ergeben. Wenn keine lange Haltbarkeit für den Motor gewünscht ist, lässt sich ein solcher Druckgasmotor leicht aus kostengünstigen Bauteilen fertigen, so dass ein Lavagesystem mit einem solchen Druckgasmotor als Wegwerfprodukt konzipiert werden kann.

Erfindungsgemäße Antriebsvorrichtungen für ein Lavagesystem können also dadurch charakterisiert sein, dass
a) ein Hohlzylinder angeordnet ist,
b) dass der Hohlzylinder an einem Ende gasdicht verschlossen ist,
c) dass der Hohlzylinder ein offenes Zylinderende hat,
d) dass ein Stab in dem Zylinder vorhanden ist,
e) dass an einem Ende des Stabs ein erstes Kolbenteil angeordnet ist, das in axialer Richtung mindestens eine durchgehende, gasdurchlässige Bohrung besitzt, und fest mit dem Stab verbunden ist,
f) dass ein zweites Kolbenteil auf dem Stab neben dem ersten Kolbenteil angeordnet ist, wobei das zweite Kolbenteil keine Gasdurchlässe enthält, und mit dem Stab fest verbunden ist,
g) dass zwischen dem ersten Kolbenteil und dem zweiten Kolbenteil ein Abstand von mindestens 1,0 mm vorhanden ist,
h) dass das zweite Kolbenteil auf der dem ersten Kolbenteil abgewandten Seite mit einer Feder kontaktiert ist, die durch eine für den Stab durchlässige Platte oder einem Steg abgestützt wird,
i) dass eine erste Öffnung einen Abstand zum verschlossenen Ende des Hohlzylinders von mindestens der Länge des ersten Kolbenteils hat,
j) dass die Feder eine solche Länge hat, dass das erste Kolbenteil gegen das verschlossene Ende des Hohlzylinders gedrückt wird, so dass die erste Öffnung nicht vom ersten Kolbenteil verdeckt wird und das zweite Kolbenteil eine zweite Öffnung verdeckt,
k) dass das erste Kolbenteil und das zweite Kolbenteil dicht an der Innenwand des Hohlzylinders anliegen,
l) dass die erste Öffnung mit einem Reservoir verbunden ist, das ein komprimiertes Gas enthält, und
m) dass die zweite Öffnung mit der Umgebung gasdurchlässig verbunden ist, wobei die Umgebung einen geringeren Druck besitzt als das komprimierte Gas im Reservoir.

Erfindungsgemäße Vorrichtungen funktionieren beispielsweise in der Weise, dass durch eine Rückstelleinrichtung, wie eine Feder, die Kolbenstange mit dem ersten und zweiten Kolbenteil gegen das verschlossene Ende des Hohlzylinders gedrückt ist. Dabei ist die erste Öffnung frei und die zweite Öffnung wird durch das zweiten Kolbenteil abgedeckt und ist damit verschlossen. Wenn komprimiertes Gas durch die erste Öffnung in den zylindrischen Innenraum geleitet wird, dann tritt das komprimierte Gas in den Zwischenraum zwischen dem ersten und zweiten Kolbenteil. Das Gas tritt dann durch den mindestens einen gasdurchlässigen Durchgang im ersten Kolbenteil bis es auf das verschlossene, gasdichte Ende des Innenraums trifft. Das Gas expandiert und drückt die beiden Kolbenteile in Richtung des Endes des Innenraums, durch das sich die Kolbenstange erstreckt. Das erste Kolbenteil bewegt sich dabei so, bis die erste Öffnung vom ersten Kolbenteil abgedeckt wird. Das zweite Kolbenteil bewegt sich synchron dazu und gibt die zweite Öffnung frei. Wenn an der zweiten Öffnung ein geringer Umgebungsdruck anliegt, dann entweicht das komprimierte Gas aus dem Bereich zwischen den beiden Kolben und dem Bereich zwischen dem ersten Kolbenteil und dem Ende des verschlossenen Innenraums über den mindestens einen gasdurchlässigen Durchgang im ersten Kolbenteil. Der Innendruck im Innenraum ist dann gleich dem Außendruck. Das Rückstellelement drückt dann auf das zweite Kolbenteil und bewegt dieses mit der Kolbenstange und dem ersten und dem zweitem Kolbenteil in die Ausgangslage zurück. Dann beginnt der Zyklus von neuem.

Wenn die beiden Kolbenteile und die Kolbenstange und gegebenenfalls die Verbindung eine geringe Masse und damit eine geringe Trägheit haben, wenn die Rückstelleinrichtung, also zum Beispiel die Feder entsprechend dimensioniert ist und der Gasdruck ausreichend ist, dann können mit der Vorrichtung hohe Impulszahlen und hohe Frequenzen erreicht werden. Die Kolbenstange kann dann die Impulse mit einem Stößel auf geeignete Membranen oder andere Übertragungsvorrichtungen abgeben, um Impulse und damit Sprühstöße mit einer Spülflüssigkeit zu erzielen.

Vorteilhaft ist auch, dass gegebenenfalls anstelle der Feder ein drittes Kolbenteil als Rückstelleinrichtung auf der Kolbenstange angeordnet ist, das fest mit der Kolbenstange verbunden ist und auch fest mit dem zweiten Kolbenteil verbunden sein kann. Das dritte Kolbenteil kann mindestens eine axiale, gasdurchlässige Bohrung enthalten. Zudem ist eine dritte Öffnung in dem Holzylinder angeordnet, die ebenfalls mit einem Reservoir verbunden ist, das ein komprimiertes Gas enthält, und eine vierte Öffnung im Hohlzylinder angeordnet ist, die mit der Umgebung verbunden ist. Bei dieser Variante erfolgt der Antriebsprozess einmal in Richtung des Stößels und dann zur Rückwärtsbewegung entgegengesetzt mit dem dritten Kolben und der dritten und vierten Öffnung.

Es ist ebenfalls erfindungsgemäß, dass am Hohlzylinder ein Befestigungsmittel für einen Lavagevorsatz angeordnet ist. Als Befestigungsmittel ist besonders ein Bajonett-Verschluss geeignet.

Vorteilhaft ist auch ein Handgriff, wobei in diesem Handgriff ein Bedienhebel angeordnet ist, der ein Ventil steuert, das mit dem Reservoir für komprimiertes Gas und mit der ersten Öffnung und gegebenenfalls mit der dritten Öffnung gasdurchlässig verbunden ist. Weiterhin ist in dem Handgriff eine Abflussöffnung für das entspannte Gas vorgesehen.

Der Druckgasmotor kann durch eine stationäre Druckluftanlage oder auch durch Gaspatronen betrieben werden. Bevorzugt ist dabei der Antrieb durch Kohlendioxidpatronen, die den Vorteil haben, dass Kohlendioxid preiswert beziehbar ist und keine Toxizität aufweist.

Im Folgenden werden Ausführungsbeispiele der Erfindung anhand von sieben schematisch dargestellten Figuren erläutert, ohne jedoch dabei die Erfindung zu beschränken. Dabei zeigt:
- Figur 1:: eine schematische Querschnittansicht eines erfindungsgemäßen Druckgasmotors mit eingefahrenem Stößel;
- Figur 2:: eine schematische Querschnittansicht des erfindungsgemäßen Druckgasmotors nach Figur 1 mit ausgefahrenem Stößel;
- Figur 3:: eine schematische Querschnittansicht eines zweiten erfindungsgemäßen Druckgasmotors mit eingefahrenem Stößel;
- Figur 4:: eine schematische Querschnittansicht des erfindungsgemäßen Druckgasmotors nach Figur 3 mit Stößel in einer Zwischenposition;
- Figur 5:: eine schematische Querschnittansicht des erfindungsgemäßen Druckgasmotors nach Figur 3 mit ausgefahrenem Stößel;
- Figur 6:: eine schematische Querschnittansicht eines erfindungsgemäßen Lavagesystems; und
- Figur 7:: eine schematische Querschnittansicht eines dritten erfindungsgemäßen Druckgasmotors.

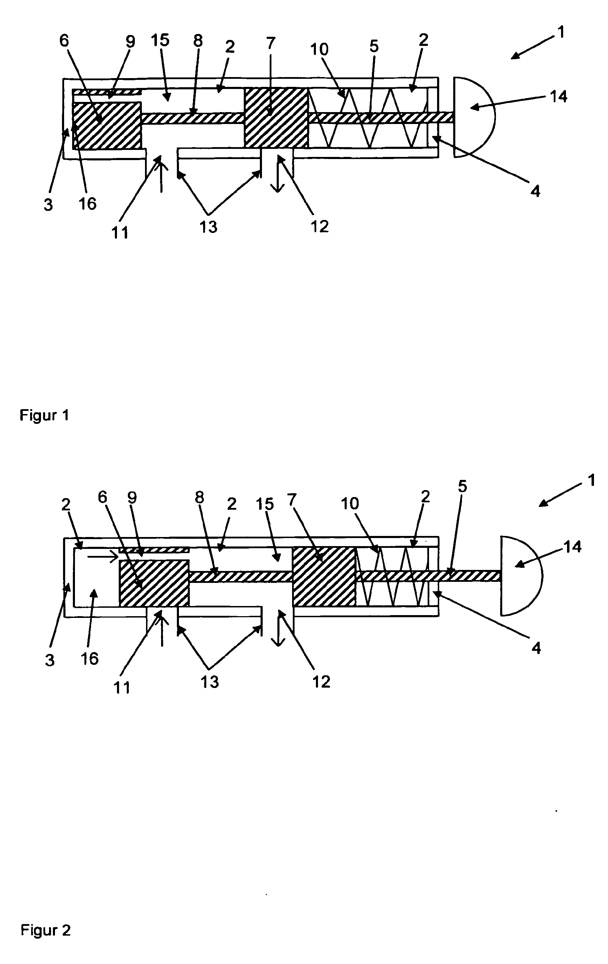

Figur 1 zeigt einen linearen Druckgasmotor 1 mit einem zylindrischen Gehäuse, das einen zylindrischen Innenraum 2 mit Innenwänden begrenzt. Die erste Grundfläche 3 des zylindrischen Innenraums 2 (In Figur 1 auf der linken Seite) ist gasdicht und druckdicht verschlossen. Die zweite Grundfläche 4 des zylindrischen Innenraums ist ebenfalls verschlossen, umfasst jedoch einen Durchgang für eine Kolbenstange 5, die sich ins Innere des Innenraums 2 erstreckt. Mit der Kolbenstange 5 verbunden sind zwei zylindrische Kolbenteile 6, 7, die im Inneren des Innenraums 2 verschiebbar, das heißt, in Längsrichtung des zylindrischen Innenraums 2 beweglich angeordnet sind. Die beiden Kolbenteile 6, 7 sind über eine Verbindungsstange 8 miteinander verbunden. Das erste Kolbenteil 6 umfasst einen Durchgang 9 in Form einer Bohrung, während das zweite Kolbenteil 7 keinen Durchgang hat. Die beiden Kolbenteile 6, 7 schließen dicht mit der Innenwand des Innenraums 2 ab. Zwischen der zweiten Grundfläche 4 des Innenraums 2 und dem zweiten Kolbenteil 7 ist eine Feder 10 angeordnet, die den Kolben 6, 7 mit der Kolbenstange 5 und der Verbindungsstange 8 in Richtung der ersten Grundfläche 3 drückt.

Das erste Kolbenteil 6 ist an der dicht verschlossenen ersten Grundfläche 3 positioniert. Diese Position des Systems Kolben 6, 7, Verbindungsstange 8 und Kolbenstange 5 wird als Ausgangsposition definiert, da dies die Position ist, die sich durch den Druck der Feder 10 ohne Anlegen eines Drucks einstellt. In den Zylindermantelwänden des Innenraums 2 sind zwei Öffnungen 11, 12 angeordnet, an denen Anschlussstücke 13 zum Anschließen eines Rohrs oder eines Schlauchs (nicht gezeigt) angeordnet sind. An der ersten Öffnung 11 soll erfindungsgemäß eine Druckgasquelle angeschlossen werden, während die zweite Öffnung 12 im einfachsten Fall einfach offen zur Umgebung ist, aber auch an eine Ableitung angeschlossen sein kann. Zur Übertragung von Stößen ist an dem aus dem Druckgasmotor 1 herausragenden Ende der Kolbenstange 5 ein Stößel 14 angeordnet. Die beiden Kolbenteile 6, 7 sind voneinander beabstandet über die Verbindungsstange 8. Zwischen den Kolbenteilen 6, 7 ist so ein Zwischenraum 15 gebildet, der sich mit der Bewegung der Kolbenteile 6, 7 im Innenraum 2 des Druckgasmotors bewegt. Zwischen dem ersten Kolbenteil 6 und der ersten Grundfläche 3 ist ein Innenraumbereich 16 eingeschlossen, der in Figur 1 keine Ausdehnung hat, da in der gezeigten Ausgangsposition das erste Kolbenteil 6 an der Grundfläche 3 anliegt.

Wenn in der in Figur 1 gezeigten Ausgangsposition des Druckgasmotors 1 ein Überdruck an der ersten Öffnung 11 angelegt wird, vergrößert sich der Druck auf die dem ersten Kolbenteil 6 zugewandte erste Seite des zweiten Kolbenteils 7. Dadurch vergrößert sich die Kraft, die auf dieser ersten Seite des zweiten Kolbenteils 7 anliegt. Als Gegenkraft wirkt der äußere Luftdruck, der auf dem Stößel 14 und auf der der Feder 10 zugewendeten zweiten Seiten des Kolbenteils 7 lastet. Zudem übt die Feder 10 eine Kraft auf diese zweite Seite aus. Wenn eine durch Druckluft oder ein anderes komprimiertes Gas aufgebrachte Kraft auf der ersten Seite des Kolbenteils 7 die Kräfte, die auf die zweite Seite des Kolbenteils wirkenden Kräfte und die Haftreibung überwindet, so wird dies zu einer Bewegung des Kolbensystems umfassend die Kolbenteile 6, 7, die Verbindungsstange 8, die Kolbenstange 5 (in Figur 1 schraffiert dargestellte Flächen) und den Stößel 14 weg von der ersten Grundfläche 3 des Innenraums 2 führen. Dabei wird die Feder 10 zusammengedrückt, wodurch die gegen die Bewegung gerichtete Kraft während der Bewegung ansteigt. Wenn die zweite Grundfläche 4 des Innenraums 2 ebenfalls gasdicht geschlossen ist, indem eine gasdichte Durchführung für die Kolbenstange 5 vorgesehen ist, bewirkt auch die in dem Teil des Innenraums 2 eingeschlossene Luft, die durch die zweite Seite des zweiten Kolbenteils 7 und durch die zweite Grundfläche 4 abgeschlossen ist, beziehungsweise das eingeschlossene Gas eine Kraft die antiproportional dem Volumen dieses Teils des Innenraums 2 ist und die der Bewegung des Kolbens entgegen gerichtet ist.

Durch den Durchlass 9 dringt die Druckluft oder das komprimierte Gas zwischen die erste Seite (in Figur 1 linke Seite) des ersten Kolbens 6 und den Abschluss der ersten Grundfläche 3 ein. Dadurch wird das Volumen im Innenraum 2 für das über die erste Öffnung 11 eingespeiste, komprimierte Gas größer. Die Bewegung wird soweit führen, dass die zweite Öffnung 12 zum Auslass, die in der Ausgangsposition durch das zweite Kolbenteil 7 verdeckt ist, geöffnet wird und das komprimierte Gas aus dem Zwischenraum 15 und dem Raum zwischen dem ersten Kolbenteil 6 und der ersten Grundfläche 3 durch die Öffnung 12 entweichen kann. Aufgrund der Trägheit der Bewegung des Kolbensystems und der Strömungswiderstände für das entweichende, komprimierte Gas kommt die Bewegung jedoch nicht sofort zum Stillstand, sondern setzt sich fort bis zu einer zweiten Position, an der sich die Bewegung umkehrt. Die genaue zweite Position ist von verschiedenen Parametern abhängig, wie der genauen Geometrie des Aufbaus, der Reibung der Kolben 6, 7 an den Innenwänden des Innenraums 2, der Federkonstanten der Feder 10, dem Druck des Gases an der ersten Öffnung 11, den Strömungswiderständen hinter der zweiten Öffnung 12 und dem Umgebungsluftdruck. Die Bewegung des Kolbensystems 5, 6, 7, 8 endet spätestens an der zweiten Grundfläche 4.

Die zweite Position, an der eine Umkehr der Bewegung des Kolbensystems 5, 6, 7, 8 stattfindet, ist in Figur 2 gezeigt. Die erste Öffnung 11 ist durch das erste Kolbenteil 6 verschlossen. Das Druckgas entweicht auch aus dem Innenraumbereich 16 zwischen dem ersten Kolbenteil 6 und der ersten Grundfläche 3 durch den Durchgang 9 (wie in Figur 2 durch den horizontalen Pfeil angedeutet) und schließlich aus der zweiten Öffnung 12, die in dieser Position nicht durch das zweite Kolbenteil 7 verschlossen ist. Dadurch fällt der Druck, der auf die erste, linke Seite des zweiten Kolbenteils 7 wirkt. Schließlich führen die Gegenkräfte durch die Feder 10 dazu, dass sich die Bewegung umkehrt und das Kolbensystem 5, 6, 7, 8 in Richtung der ersten Grundfläche 3 beschleunigt wird. Durch die Bewegung in Richtung der ersten Grundfläche 3 nimmt die Federkraft ab. Gleichzeitig wird die zweite Öffnung 12 durch den zweiten Kolbenteil 7 verschlossen und die erste Öffnung 11 wieder geöffnet, so dass sich erneut ein Druck im Zwischenraum 15 und in dem Innenraumbereich 16 zwischen dem ersten Kolbenteil 6 und der ersten Grundfläche 3 des Innenraums aufbauen kann. Das Kolbensystem 5, 6, 7, 8 kehrt zurück in die Ausgangsposition nach Figur 1 und die Bewegung beginnt von vorne.

Solange an der ersten Öffnung 11 ein komprimiertes Gas angelegt und so zyklisch eingespeist wird und die zweite Öffnung 12 zu einem Normaldruckbereich oder Unterdruckbereich offen bleibt, wird die Bewegung zyklisch fortgesetzt. Die resultierende Bewegung des Stößels 14 kann dazu genutzt werden, Sprühstöße einer Spülflüssigkeit zu erzeugen, indem der Stößel 14 beispielsweise auf eine Membran schlägt, die den Druckstoß an die dahinter gelagerte Spülflüssigkeit weitergibt, die dann durch eine oder mehrere Düsen austreten kann.

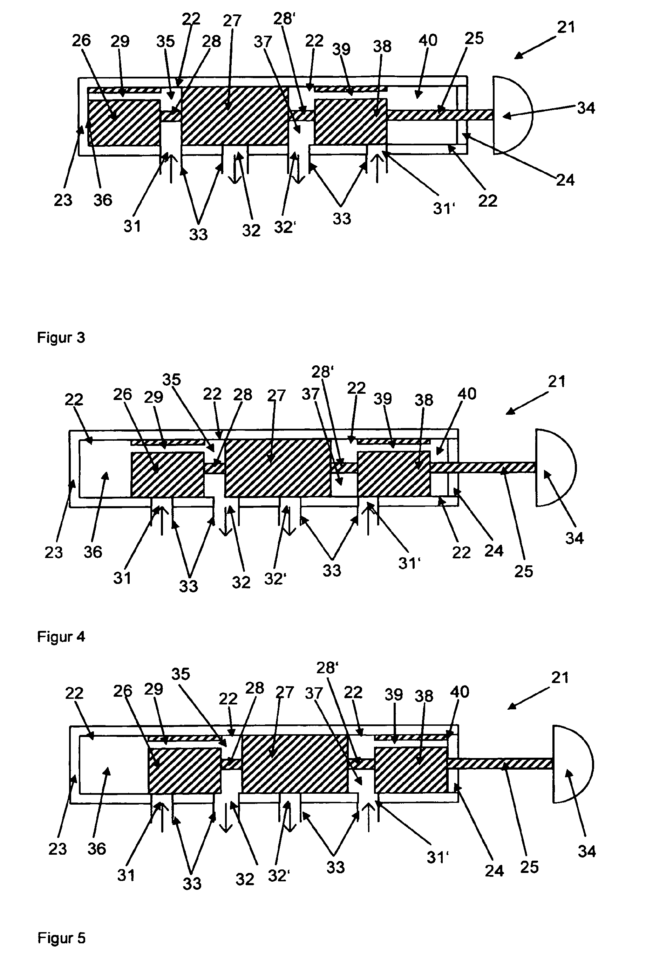

Die Figuren 3, 4 und 5 zeigen schematische Querschnittansichten eines alternativen Druckgasmotors 21, bei welchem als Rückstelleinrichtung keine Feder, wie im vorigen Ausführungsbeispiel, zum Einsatz kommt, sondern ein zusätzlicher Kolbenantrieb.

Ein zylindrischer Innenraum 22 des Druckgasmotors 21 ist an einer ersten Grundfläche 23 und einer zweiten Grundfläche 24 gasdicht verschlossen. Eine Kolbenstange 25 ragt durch eine gasdichte Öffnung in der zweiten Grundfläche 24 aus dem Innenraum 22 heraus. Die Kolbenstange 25 ist in Richtung der Zylinderachse des Innenraums 22 beweglich durch die zweite Grundfläche 24 angeordnet.

An der Kolbenstange 25 sind drei Kolbenteile 26, 27, 38 angeordnet. Das erste Kolbenteil 26 ist an dem der zweiten Grundfläche 24 gegenüberliegenden Ende der Kolbensystems 25, 26, 27, 28, 28', 38 angeordnet. Das zweite Kolbenteil 27 ist beabstandet von dem ersten Kolbenteil 26 über eine Verbindungsstange 28 mit dem ersten Kolbenteil 26 verbunden. Im ersten Kolbenteil 26 ist ein Durchgang 29 in Form einer Bohrung angeordnet.

In der Zylindermantelwand des Innenraums 22 sind eine erste Öffnung 31 und eine vierte Öffnung 31' zum Einspeisen eines komprimierten Gases sowie eine zweite Öffnung 32 und eine dritte Öffnung 32' zum Ableiten eines Gases angeordnet. An den Öffnungen 31, 31', 32, 32' sind Anschlussstücke 33 zum Anschließen eines Schlauchs oder Rohrs angeordnet.

An dem durch die zweite Grundfläche 24 aus dem Druckgasmotor 21 herausragenden Ende der Kolbenstange 25 ist ein Stößel 34 in Form eines Pilzes befestigt. Der Stößel 34 dient dazu, bei einer oszillierenden Bewegung des Kolbensystems 25, 26, 27, 28, 28' 38 im Innenraum 22, bei der in Figur 5 gezeigten voll ausgefahrenen Position einen Stoß auf ein Flüssigkeitsreservoir auszuüben, um Sprühstöße für ein Lavage-System zu erzeugen.

Zwischen dem ersten Kolbenteil 26 und dem zweiten Kolbenteil 27 ist durch die Beabstandung ein erster Zwischenraum 35 im Innenraum 22 gebildet. Zwischen dem ersten Kolbenteil 26 und der ersten Grundfläche 23 ist ein erster Innenraumbereich 36 ausgebildet, der in Figur 3 keine Ausdehnung hat und sich bei anderen Positionen des Kolbensystems 25, 26, 27, 28, 28', 38 nach Figur 4 und 5 öffnet. Ein zweiter Zwischenraum 37 ist zwischen dem zweiten Kolbenteil 27 und einem dritten Kolbenteil 38 angeordnet. Das dritte Kolbenteil 38 umfasst einen Durchgang 39 und ist grundsätzlich wie das erste Kolbenteil 26 aufgebaut. Das dritte Kolbenteil 38 ist über eine Verbindungstange 28' mit dem zweiten Kolbenteil 27 verbunden. Zwischen dem dritten Kolbenteil 38 und der zweiten geschlossenen Grundfläche 24 ist ein zweiter Innenraumbereich 40 ausgebildet, der in der Ausgangsposition (Figur 3) des Kolbensystems 25, 26, 27, 28, 28', 38 maximales Volumen hat, während er in der Endposition (Figur 5) kein Volumen mehr aufweist. Im Gegensatz zu den variablen Volumina der beiden Innenraumbereiche 36, 40 haben die Zwischenräume 35, 37 fest vorgegebene Volumina innerhalb des Innenraums 22.

Das erste Kolbenteil 26, das zweite Kolbenteil 27, das dritte Kolbenteil 38, und die Verbindungsstangen 28, 28' bilden einen Kolben 26, 27, 28, 28', 38, der im Inneren des zylindrischen Innenraums 22 in Richtung der Zylinderachse beweglich ist. Der zweite Zwischenraum 37, das dritte Kolbenteil 38 mit dem Durchgang 39, der zweite Innenraumbereich 40 und die gasdicht verschlossene zweite Grundfläche 24 bilden zusammen mit der dritten Öffnung 32' und der vierten Öffnung 31' bei dieser Ausführungsform eine Rückstelleinrichtung für das Kolbensystem 25, 26, 27, 28, 28', 38.

Die Kolbenteile 26, 27, 38 schließen mit den Zylindermantelwänden des Innenraums 22 dicht ab und werden darin geführt. Abhängig von der Position des Kolbens 26, 27, 28, 28', 38 kann das erste Kolbenteil 26 die erste Öffnung 31 zum Einspeisen eines komprimierten Gases bedecken oder offen lassen, das dritte Kolbenteil 38 kann die vierte Öffnung 31' zum Einspeisen eines komprimierten Gases bedecken oder offen lassen und das zweite Kolbenteil 27 kann die zweite Öffnung 32 und die dritte Öffnung 32' zum Auslassen des Gases bedecken oder eine von beiden offen lassen. Es kann immer nur eine der beiden Einlassöffnungen 31, 31' und eine der beiden Auslassöffnungen 32, 32' geöffnet sein. Wenn die erste Öffnung 31 zum Einspeisen eines komprimierten Gases geöffnet ist, so ist die zweite Öffnung 32 zum Ablassen des dann expandierten Gases geschlossen. Das gleiche gilt für die anderen beiden Öffnungen 31' und 32'. Durch das zweite Kolbenstück 27 sind eine erste Gaskolbenkammer 29, 35, 36 und eine zweite Gaskolbenkamer 37, 39, 40 voneinander getrennt gebildet und bilden so über die Durchgänge 29 und 39 separate Gaskolbenkammern beziehungsweise Motorteile.

In der in Figur 3 gezeigten Ausgangsposition strömt ein komprimiertes Gas durch die Öffnung 31 in den ersten Zwischenraum 35 und in den Durchgang 29 des ersten Kolbenteils 26. Dort drückt das Gas auf die erste Grundfläche 23. Gleichzeitig entweicht ein Gas aus dem zweiten Zwischenraum 37 und über den Durchgang 39 des dritten Kolbenteils 38 auch aus dem zweiten Innenbereich 40 durch die dritte Öffnung 32'. Da der Druck in der ersten Gaskolbenkammer 29, 35, 36 größer als der in der zweiten Gaskolbenkamer 37, 39, 40 wird, wird der Kolben 26, 27, 28, 28', 38 in Richtung der zweiten Grundfläche 24 bewegt, so dass die Kolbenstange 25 aus dem Innenraum 22 des Druckgasmotors 21 heraus getrieben wird.

Durch die Bewegung des ersten Kolbenteils 26 wird die erste Öffnung 31 geschlossen und danach, bei einer weiteren Bewegung des Kolbens 26, 27, 28, 28', 38, die zweite Öffnung 32 geöffnet. Dies führt dazu, dass sich der Druck in der ersten Gaskolbenkammer 29, 35, 36 abbaut. Parallel dazu wird die dritte Öffnung 32' durch das zweite Kolbenteil 27 geschlossen, wodurch sich durch die Verringerung des Volumens des zweiten Innenraumbereichs 40 ein Druck in der zweiten Gaskolbenkamer 37, 39, 40 aufbaut. Diese Situation ist in Figur 4 dargestellt. Durch die Zeit, die es benötigt, den Druck in der ersten Gaskolbenkammer 29, 35, 36 abzubauen und durch die Trägheit des Kolbensystems 25, 26, 27, 28, 28', 38 mit dem Stößel 34 bewegt sich der Kolben 26, 27, 28, 28', 38 jedoch weiter bis zu der in Figur 5 dargestellten Position. Dort endet die Bewegung des Kolbens 26, 27, 28, 28', 38 an der zweiten Grundfläche 24 und die vierte Öffnung 31' öffnet sich, während die zweite Öffnung 32 noch weit geöffnet ist. Dadurch strömt das komprimierte Gas durch die vierte Öffnung in die zweite Gaskolbenkammer 37, 39, 40 und baut dort einen Druck auf, während das expandierte Gas aus der ersten Gaskolbenkammer 29, 35, 36 durch die zweite Öffnung 32 entweicht. Dadurch, dass sich ein Druckunterschied mit umgekehrtem Vorzeichen in den beiden Gaskolbenkammern 29, 35, 36 und 37, 39, 40 aufbaut, wird der Kolben 26, 27, 28, 28', 38 nun in Richtung der ersten Grundfläche 23 beschleunigt.

Der Kolben kehrt über die in Figur 4 dargestellte Position hinweg zur Ausgangsposition nach Figur 3 zurück. Die Trägheit des Kolbens 26, 27, 28, 28', 38 bei der entgegengesetzt gerichteten Bewegung und der sich nur langsam abbauende Druckunterschied bewirken, dass der Kolben 26, 27, 28, 28', 38 wieder in die Ausgangsposition zurückkehrt. Die Bewegung des Kolbens 26, 27, 28, 28', 38 setzt sich dann zyklisch fort.

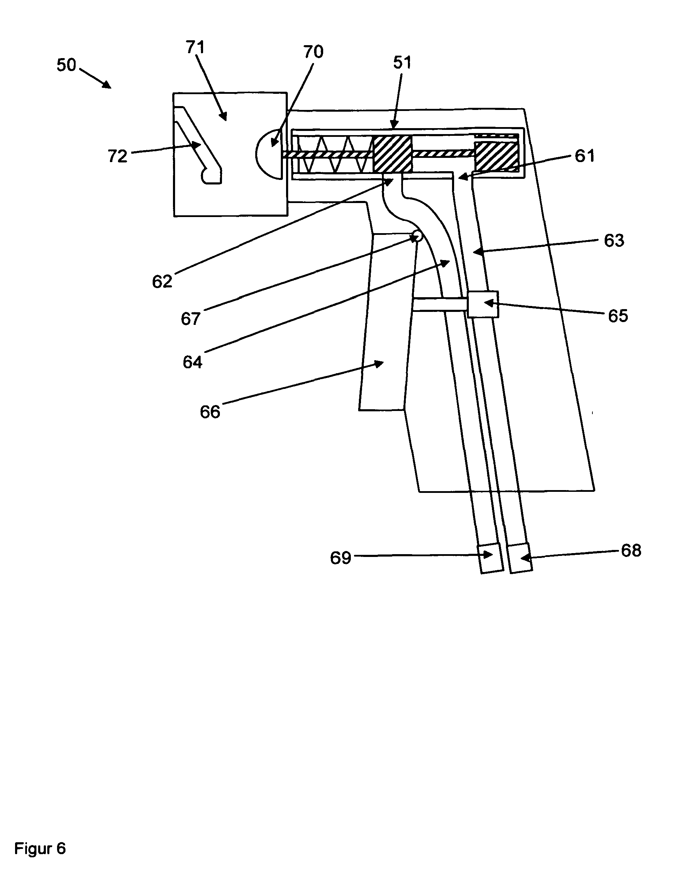

Figur 6 zeigt den schematischen Aufbau eines erfindungsgemäßen Handgriffs 50 für ein Lavagesystem in Querschnittansicht. Ein erfindungsgemäßer Druckgasmotor 51 ist in dem Handgriff 50 angeordnet. Der Druckgasmotor umfasst eine erste Öffnung 61 zum Einspeisen von Druckluft und eine zweite Öffnung 62 zum Ablassen von Gas. Die beiden Öffnungen 61, 62 sind an eine Druckluftleitung 63 und eine Abluftleitung 64 angeschlossen.

In der Druckluftleitung 63 ist ein Ventil 65 angeordnet. Das Ventil 65 ist manuell über eine Bedieneinrichtung in Form eines Bedienhebels 66, der drehbar über ein Gelenk 67 mit dem Handgriff 50 verbunden ist, bedienbar. Die Druckluftleitung 63 umfasst einen Anschluss 68, über den sie an eine Druckluftquelle anschließbar ist. Die Abluftleitung 64 mündet in die Umgebung oder ist über einen zweiten Anschluss 69 mit einem Abluftsystem oder sogar einer Vakuumpumpe zum Erzeugen eines Unterdrucks verbunden.

Im Bereich eines Stößels 70, der am Druckgasmotor 51 angebracht ist, ist eine Halterung 71 mit einem Bajonettverschluss 72 für ein Lavagesystem (nicht gezeigt) angeordnet. Ein Lavagesystem kann über die Halterung 71 und den Bajonettverschluss 72 mit dem Handgriff 50 verbunden werden. Durch Betätigen des Bedienhebels 66 kann ein Anwender das Ventil 65 bedienen und so den Druckgasmotor 51 über die Druckluftleitung 63 mit Druckluft versorgen. Dadurch bewegt sich der Druckgasmotor 51, so dass der Stößel 70 mit hoher Frequenz auf ein Flüssigkeitsreservoir des Lavagesystems schlägt. Dadurch werden Sprühstöße mit hoher Frequenz erzeugt, mit der sich Gewebeareale bei einem Patienten reinigen lassen.

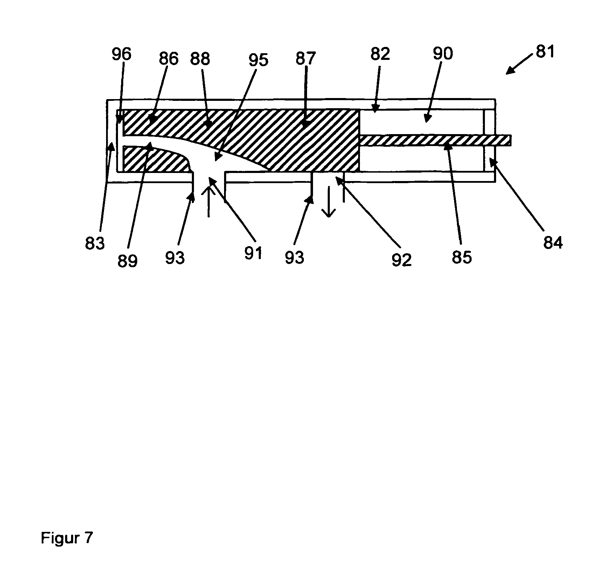

Figur 7 zeigt eine dritte Ausführungsform eines erfindungsgemäßen Druckgasmotors 81 in schematischer Querschnittansicht. Ein zylindrischer Innenraum 82 ist an beiden Grundflächen 83, 84 gasdicht abgeschlossen. Die zweite Grundfläche 84 umfasst eine Öffnung für eine Kolbenstange 85. Die Kolbenstange 85 ist fest und unbeweglich an einem, aus einem ersten Kolbenteil 86, einem zweiten Kolbenteil 87 und einer Verbindung 88 gebildeten Kolben 86, 87, 88 befestigt. Der Kolben 86, 87, 88 und die Kolbenstange 85 sind in Längsrichtung des Innenraums 82 beweglich.

Im ersten Kolbenteil 86 ist ein Durchgang 89 angeordnet. Da der Kolben 86, 87, 88 dicht mit den Innenwänden des Innenraums 82 abschließt, bildet der Bereich des Innenraums 22, der durch das zweite Kolbenteil 87 und die zweite Grundfläche gasdicht abgeschlossen ist, eine Gasfeder 90. Die Gasfeder 90 wirkt als Rückstelleinrichtung für den Gasdruckmotor 81.

Im Zylindermantel des Innenraums 82 sind eine erste Öffnung 91 zum Einspeisen eines komprimierten Gases und eine zweite Öffnung 92 zum Ablassen eines Gases vorgesehen. An den Öffnungen sind Anschlüsse 93 zum Anschließen von Leitungen angeordnet. Im Bereich der Verbindung 88 bildet der Kolben 86, 87, 88 einen Zwischenraum 95 zwischen dem ersten Kolbenteil 86 und dem zweiten Kolbenteil 87. Zwischen dem ersten Kolbenteil 86 und der ersten Grundfläche 83 wird ein Innenraumbereich 96 gebildet, dessen Volumen von der Position des Kolbens 86, 87, 88 im Innenraum 82 der Druckgasmotors 81 abhängt.

Der Druck in der Gasfeder 90 muss so gewählt werden, dass er kleiner als der des durch die erste Öffnung 91 eingespeisten komprimierten Gases ist. Der Druck in der Gasfeder 90 wird bevorzugt größer als der Umgebungsdruck gewählt. Die periodische Bewegung des Gasdruckmotors 81 läuft nach dem beim ersten Ausführungsbeispiel nach den Figuren 1 und 2 gezeigten Muster ab.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 21, 51, 81: Druckgasmotor
- 2, 22, 82: Innenraum
- 3, 23, 83: erste Grundfläche
- 4, 24, 84: zweite Grundfläche
- 5, 25, 85: Kolbenstange
- 6, 26, 86: erstes Kolbenteil
- 7, 27, 87: zweites Kolbenteil
- 8, 28, 28': Verbindungsstange
- 9, 29, 39, 89: Durchgang / Bohrung
- 10, 90: Feder
- 11, 31, 61, 91: erste Öffnung
- 12, 32, 62, 92: zweite Öffnung
- 13, 33, 93: Anschlussstück / Hülse
- 14, 34, 70: Stößel
- 15, 35, 37, 95: Zwischenraum
- 16, 36, 40, 96: Innenraumbereich
- 31': vierte Öffnung
- 32': dritte Öffnung
- 38: drittes Kolbenteil
- 50: Handgriff
- 63: Druckluftleitung
- 64: Abluftleitung
- 65: Ventil
- 66: Bedienhebel
- 67: Gelenk
- 68, 69: Anschluss
- 71: Halterung
- 72: Bajonettverschluss
- 88: Verbindung

## Patentansprüche

1. Druckgasmotor (1, 21, 51, 81) zum Betreiben eines Lavagesystems umfassend
einen zylindrischen Innenraum (2, 22, 82), der an zumindest einer ersten Grundfläche (3, 23, 83) gasdicht verschlossen ist,
zumindest einen Kolben, der beweglich entlang der Zylinderachse im zylindrischen Innenraum (2, 22, 82) angeordnet ist und der dicht mit dem Innenraum (2, 22, 82) schließt, eine Kolbenstange (5, 25, 85), die mit dem Kolben verbunden ist und die über eine zweite Grundfläche (4, 24, 84) aus dem Innenraum (2, 22, 82) hinaus ragt,
eine erste Öffnung (11, 31, 61, 91) zum Einspeisen eines komprimierten Gases in den Innenraum (2, 22, 82) und
eine zweite Öffnung (12, 32, 62, 92) zum Ableiten eines Gases aus dem Inneren des Innenraums (2, 22, 82),
**dadurch gekennzeichnet, dass**
die Öffnungen (11, 12, 31, 32, 61, 62, 91, 92) im Zylindermantel des zylindrischen Innenraums (2, 22, 82) angeordnet sind,
wobei die erste Öffnung (11, 31, 61, 91) zwischen der zweiten Öffnung (12, 32, 62, 92) und der verschlossenen ersten Grundfläche (3, 23, 83) angeordnet ist,
der Kolben ein erstes Kolbenteil (6, 26, 86) und ein zweites Kolbenteil (7, 27, 87) umfasst, die miteinander verbunden sind,
wobei das erste Kolbenteil (6, 26, 86) zumindest einen Durchgang (9, 29, 89) umfasst und/oder zwischen dem ersten Kolbenteil (6, 26, 86) und der Zylinderwand des Innenraums (2, 22, 82) zumindest einen Durchgang bildet und
das zweite Kolbenteil (7, 27, 87) das Innere des Innenraums (2, 22, 82) dicht schließt, wobei zumindest ein Durchgang (9, 29, 89) des ersten Kolbenteils (6, 26, 86) einen Zwischenraum (15, 35, 95) zwischen dem ersten und dem zweiten Kolbenteil (7, 27, 87) mit einem Innenraumbereich (16, 36, 96) zwischen der verschlossenen ersten Grundfläche (3, 23, 83) und dem ersten Kolbenteil (6, 26, 86) verbindet und
wobei die erste Öffnung (11, 31, 61, 91) durch das erste Kolbenteil (6, 26, 86) und die zweite Öffnung (12, 32, 62, 92) durch das zweite Kolbenteil (7, 27, 87) verschließbar sind und
der Druckgasmotor (1, 21, 51, 81) eine Rückstelleinrichtung (10, 90) umfasst, die zumindest zeitweise eine Kraft in Richtung der ersten Grundfläche (3, 23, 83) des zylindrischen Innenraums (2, 22, 82) auf den Kolben ausübt, wenn das zweite Kolbenteil (7, 27, 87) die zweite Öffnung (12, 32, 62, 92) nicht vollständig verschließt.

2. Druckgasmotor (1, 21, 51, 81) nach Anspruch 1, **dadurch gekennzeichnet, dass** die Wand des Innenraums (2, 22, 82) eine Furche umfasst, die sich von dem Bereich des Abschlusses der ersten Grundfläche (3, 23, 83) des Innenraums (2, 22, 82) bis zum Bereich der Höhe der zweiten Öffnung (12, 32, 62, 92) erstreckt und die zumindest einen Durchgang zwischen dem ersten Kolbenteil (6, 26, 86) und der Zylinderwand des Innenraums (2, 22, 82) bildet, wobei die Furche nicht mit den Öffnungen (11, 12, 31, 32, 61, 62, 91, 92) verbunden ist.

3. Druckgasmotor (1, 21, 51, 81) nach Anspruch 2, **dadurch gekennzeichnet, dass** die Furche sich bis in den Abschluss der ersten Grundfläche (3, 23, 83) des zylindrischen Innenraums (2, 22, 82) erstreckt und das vorzugsweise das erste Kolbenteil (6, 26, 86) ein zylindrischer Körper ist, der besonders bevorzugt keine weiteren Durchgänge (9, 29, 89) umfasst.

4. Druckgasmotor (1, 21, 51, 81) nach einem der vorangehenden Ansprüche, **dadurch gekennzeichnet, dass**
das erste Kolbenteil (6, 26, 86) und das zweite Kolbenteil (7, 27, 87) über eine starre Verbindung (8, 28, 28', 88), bevorzugt über eine Stange (8, 28, 28'), besonders bevorzugt über eine Verlängerung der Kolbenstange (5, 25, 85), insbesondere entlang der Zylinderachse, miteinander verbunden sind.

5. Druckgasmotor (1, 21, 51, 81) nach einem der vorangehenden Ansprüche, **dadurch gekennzeichnet, dass**
die zweite Grundfläche (4, 24, 84) des zylindrischen Innenraums (2, 22, 82) offen ist.

6. Druckgasmotor (1, 21, 51, 81) nach einem der vorangehenden Ansprüche, **dadurch gekennzeichnet, dass**
an der zweiten Grundfläche (4, 24, 84) des zylindrischen Innenraums (2, 22, 82) eine Halterung für die Kolbenstange (5, 25, 85) angeordnet ist, die die Kolbenstange (5, 25, 85) vorzugsweise zentrisch im Bereich der Zylinderachse des Innenraums (2, 22, 82) lagert.

7. Druckgasmotor (1, 21, 51, 81) nach einem der vorangehenden Ansprüche, **dadurch gekennzeichnet, dass**
der zumindest eine Durchgang (9, 29, 89) zumindest eine durchgehende, gasdurchlässige Bohrung (9, 29) im ersten Kolbenteil (6, 26, 86) umfasst, insbesondere eine solche Bohrung (9, 29) ist.

8. Druckgasmotor (1, 21, 51, 81) nach einem der vorangehenden Ansprüche, **dadurch gekennzeichnet, dass**
der Abstand zwischen dem ersten Kolbenteil (6, 26, 86) und dem zweiten Kolbenteil (7, 27, 87) größer als 1 mm, bevorzugt zwischen 1 mm und 100 mm ist, besonders bevorzugt zwischen 5 mm und 10 mm ist.

9. Druckgasmotor (1, 21, 51, 81) nach einem der vorangehenden Ansprüche, **dadurch gekennzeichnet, dass**
der Abstand der ersten Öffnung (11, 31, 61, 91) zur verschlossenen ersten Grundfläche (3, 23, 83) mindestens so groß ist wie die Höhe des ersten Kolbenteils (6, 26, 86) in Richtung der Zylinderachse des Innenraums (2, 22, 82).

10. Druckgasmotor (1, 21, 51, 81) nach einem der vorangehenden Ansprüche, **dadurch gekennzeichnet, dass**
das erste Kolbenteil (6, 26, 86) und/oder das zweite Kolbenteil (7, 27, 87) zumindest bereichsweise zylindrisch sind und dicht an der Innenwand des zylindrischen Innenraums (2, 22, 82) anliegen.

11. Druckgasmotor (1, 21, 51, 81) nach einem der vorangehenden Ansprüche, **dadurch gekennzeichnet, dass**
an der ersten Öffnung (11, 31, 61, 91) ein Reservoir angeschlossen ist, das ein komprimiertes Gas beinhaltet und/oder die zweite Öffnung (12, 32, 62, 92) gasdurchlässig mit der Umgebung des Druckgasmotors (1, 21, 51, 81) verbunden ist, vorzugsweise ohne den Gasstrom behindernde Leitungen (64).

12. Druckgasmotor (1, 21, 51, 81) nach einem der vorangehenden Ansprüche, **dadurch gekennzeichnet, dass**
außen im Bereich der zweiten Grundfläche (4, 24, 84) ein Befestigungsmittel (71, 72) zum Befestigen eines Lavagevorsatzes, insbesondere ein Bajonettverschluss (72), angeordnet ist.

13. Druckgasmotor (1, 21, 51, 81) nach einem der vorangehenden Ansprüche, **dadurch gekennzeichnet, dass**
das aus der zweiten Grundfläche (4, 24, 84) herausragenden Ende der Kolbenstange (5, 25, 85) einen Stößel (14, 34, 70), insbesondere einen pilzförmigen Stößel (14, 34, 70), umfasst.

14. Druckgasmotor (1, 21, 51, 81) nach Anspruch 13, **dadurch gekennzeichnet, dass**
die Kolbenteile (6, 7, 26, 27, 86, 87), die Verbindungen (8, 28, 28', 88) der Kolbenteile (6, 7, 26, 27, 86, 87), die Kolbenstange (5, 25, 85), die Wände des zylindrischen Innenraums (2, 22, 82), der oder die Abschlüsse der Grundflächen (3, 4, 23, 24, 83, 84), die Halterung, die Rückstelleinrichtung (10, 90), die Befestigungsmittel (71, 72) und/oder der Stößel (14, 34, 70) aus Kunststoff gefertigt sind, insbesondere Spritzgießteile sind.

15. Druckgasmotor (1, 21, 51, 81) nach einem der vorangehenden Ansprüche, **dadurch gekennzeichnet, dass**
der Innenraum (2, 22, 82) das Innere eines zylindrischen Rohrs ist und das der Druckgasmotor (1, 21, 51, 81) zumindest bereichsweise, vorzugsweise im Wesentlichen, eine zylindrische Außenfläche umfasst.

16. Druckgasmotor (1, 21, 51, 81) nach einem der vorangehenden Ansprüche, **dadurch gekennzeichnet, dass**
bei einer oszillierenden Bewegung des Kolbens im Innenraum (2, 22, 82) die erste Öffnung (11, 31, 61, 91) durch das erste Kolbenteil (6, 26, 86) und die zweite Öffnung (12, 32, 62, 92) durch das zweite Kolbenteil (7, 27, 87) abwechselnd vollständig verschließbar sind.

17. Druckgasmotor (1, 21, 51, 81) nach einem der vorangehenden Ansprüche, **dadurch gekennzeichnet, dass**
die Rückstelleinrichtung (10, 90) eine Feder, insbesondere eine Plastik-, Stahlfeder (10) und/oder Gasfeder (90) umfasst, die zwischen dem zweiten Kolbenteil (7, 27, 87) und der zweiten Grundfläche (4, 24, 84) des zylindrischen Innenraums (2, 22, 82) angeordnet ist.

18. Druckgasmotor (1, 21, 51, 81) nach einem der Ansprüche 1 bis 16, **dadurch gekennzeichnet, dass**
die Rückstelleinrichtung einen dritten Kolbenteil (38),
eine dritte Öffnung (32') zum Ableiten eines Gases aus dem Inneren des Innenraums (22) und
eine vierte Öffnung (31') zum Einspeisen eines komprimierten Gases in den Innenraum (22) umfasst,
wobei die dritte und vierte Öffnung (32', 31') im Zylindermantel des zylindrischen Innenraums (22) angeordnet sind,
die vierte Öffnung (31') zwischen der zweiten Öffnung (32) und der zweiten Grundfläche (24) des zylindrischen Innenraums (22) angeordnet ist,
die dritte Öffnung (32') zwischen der zweiten Öffnung (32) und der vierten Öffnung (31') angeordnet ist,
die dritte Öffnung (32') durch das zweite Kolbenteil (27) und die vierte Öffnung (31') durch das dritte Kolbenteil (38) verschließbar sind,
und wobei das dritte Kolbenteil (38) ein Teil des Kolbens ist, das an der Kolbenstange (25) zwischen dem zweiten Kolbenteil (27) und der zweiten Grundfläche (24) des zylindrischen Innenraums (22) angeordnet und mit dem zweiten Kolbenteil (27) verbunden ist, und
das dritte Kolbenteil (38) zumindest einen Durchgang (39) umfasst und/oder zwischen dem dritten Kolbenteil (38) und der Zylinderwand des Innenraums (22) zumindest einen Durchgang bildet.

19. Druckgasmotor (1, 21, 51, 81) nach Anspruch 18, **dadurch gekennzeichnet, dass**
das dritte Kolbenteil (38) wie das erste Kolbenteil (2, 26, 86) nach einem der vorangehenden Ansprüche aufgebaut ist, und/oder die Innenwand des Innenraums (2, 22, 82) in Bezug auf das dritte Kolbenteil (38) so aufgebaut ist wie in Bezug auf das erste Kolbenteil (2, 26, 86) nach einem der Ansprüche 2 oder 3.

20. Druckgasmotor (1, 21, 51, 81) nach Anspruch 18 oder 19, **dadurch gekennzeichnet, dass**
die Kolbenstange (5, 25, 85) starr und unbeweglich mit dem Kolben verbunden ist, vorzugsweise mit dem zweiten Kolbenteil (7, 27, 87) oder dem dritten Kolbenteil (38) verbunden ist, insbesondere im Bereich der Zylinderachse.

21. Handgriff (50) umfassend einen Druckgasmotor (1, 21, 51, 81) nach einem der vorangehenden Ansprüche und ein Ventil (65) und eine am Handgriff (50) angeordnete Bedieneinrichtung (66) zur Steuerung des Ventils (65), wobei das Ventil (65) in einer Leitung (63, 64), insbesondere einer Druckgasleitung (63), angeordnet ist, die mit der ersten Öffnung (11, 31, 61, 91) und/oder zweiten Öffnung (12, 32, 62, 92), vorzugsweise mit der ersten (11, 31, 61, 91) und besonders bevorzugt mit der ersten (11, 31, 61, 91) und vierten Öffnung (31'), verbunden ist, wobei der Handgriff für ein Lavagesystem vorgesehen ist.

22. Lavagesystem umfassend einen Druckgasmotor (1, 21, 51, 81) nach einem der Ansprüche 13 bis 20, bevorzugt umfassend einen Handgriff (50) nach Anspruch 21, des Weiteren umfassend ein Spülflüssigkeitsreservoir und/oder einen Anschluss für ein Spülflüssigkeitsreservoir, wobei über das aus der zweiten Grundfläche (4, 24, 84) herausragende Ende der Kolbenstange (5, 25, 85), insbesondere über den Stößel (14, 34, 70), Kraftstöße auf eine Kraftübertragungsvorrichtung, vorzugsweise eine Membran, übertragbar sind, um Sprühstöße mit der Spülflüssigkeit zu erzeugen.

23. Verfahren zum Betreiben eines Druckgasmotors nach einem der Ansprüche 1 bis 20, **gekennzeichnet durch**
Einspeisen eines komprimierten Gases **durch** die erste Öffnung in einen Zwischenraum zwischen dem ersten und dem zweiten Kolbenteil,
Aufbauen eines Überdrucks in dem Zwischenraum,
Bewegung der Kolbenteile im Innenraum des Druckgasmotors aufgrund des Überdrucks, Schließen der ersten Öffnung und Öffnen der zweiten Öffnung **durch** die Bewegung der Kolbenteile,
Ablassen des expandierten Gases **durch** die zweite Öffnung aus dem Zwischenraum, Rückstellen der Kolbenteile im Innenraum **durch** eine Rückstelleinrichtung.

## Claims

1. A compressed gas motor (1, 21, 51, 81) for operating a lavage system, comprising a cylindrical internal space (2, 22, 82) that is closed in gas-tight manner at least at one first base surface (3, 23, 83),
at least one plunger arranged in the cylindrical internal space (2, 22, 82) so as to be mobile along the cylinder axis and ending tightly against the internal space (2, 22, 82),a plunger rod (5, 25, 85) connected to the plunger and projecting from the internal space (2, 22, 82) beyond a second base surface (4, 24, 84)
a first opening (11, 31, 61, 91) for supplying a compressed gas into the internal space (2, 22, 82), and
a second opening (12, 32, 62, 92) for discharging a gas from the inside of the internal space (2, 22, 82),
**characterised in that**
the openings (11, 12, 31, 32, 61, 62, 91, 92) are arranged in the cylinder jacket of the cylindrical internal space (2, 22, 82),
wherein the first opening (11, 31, 61, 91) is arranged between the second opening (12, 32, 62, 92) and the closed first base surface (3, 23, 83),
the plunger comprises a first plunger part (6, 26, 86) and a second plunger part (7, 27, 87) that are connected to each other;
wherein the first plunger part (6, 26, 86) comprises at least one passage (9, 29, 89) and/or forms at least one passage between the first plunger part (6, 26, 86) and the cylinder wall of the internal space (2, 22, 82), and
the second plunger part (7, 27, 87) closes the inside of the internal space (2, 22, 82) tightly,
wherein at least one passage (9, 29, 89) of the first plunger part (6, 26, 86) connects an intermediate space (15, 35, 95) between the first and the second plunger part (7, 27, 87) to an internal space region (16, 36, 96) between the closed first base surface (3, 23, 83) and the first plunger part (6, 26, 86), and
wherein the first opening (11, 31, 61, 91) can be closed by the first plunger part (6, 26, 86) and the second opening (12, 32, 62, 92) can be closed by the second plunger part (7, 27, 87), and
the compressed gas motor (1, 21, 51, 81) comprises a restoring arrangement (10, 90) that exerts, at least for part of the time, a force on the plunger in a direction of the first base surface (3, 23, 83) of the cylindrical internal space (2, 22, 82) when the second plunger part (7, 27, 87) does not close the second opening (12, 32, 62, 92) completely.

2. The compressed gas motor (1, 21, 51, 81) according to Claim 1, **characterised in that** the wall of the internal space (2, 22, 82) comprises a groove extending from the region of the end of the first base surface (3, 23, 83) of the internal space (2, 22, 82) to the region of the height of the second opening (12, 32, 62, 92) and forming at least one passage between the first plunger part (6, 26, 86) and the cylinder wall of the internal space (2, 22, 82), wherein the groove is not connected to the openings (11, 12, 31, 32, 61, 62, 91, 92).

3. The compressed gas motor (1, 21, 51, 81) according to Claim 2, **characterised in that** the groove extends up to the end of the first base surface (3, 23, 83) of the cylindrical internal space (2, 22, 82) and the first plunger part (6, 26, 86) is a cylindrical body particularly preferably comprising no further passages (9, 29, 89).

4. The compressed gas motor (1, 21, 51, 81) according to one of the preceding claims,
**characterised in that**
the first plunger part (6, 26, 86) and the second plunger part (7, 27, 87) are connected to each other by means of a rigid connection (8, 28, 28', 88), preferably by means of a rod (8, 28, 28'), particularly preferably by means of an extension of the plunger part (5, 25, 85), in particular along the cylinder axis.

5. The compressed gas motor (1, 21, 51, 81) according to one of the preceding claims,
**characterised in that**
the second base surface (4, 24, 84) of the cylindrical internal space (2, 22, 82) is open.

6. The compressed gas motor (1, 21, 51, 81) according to one of the preceding claims,
**characterised in that**
a bracket for the plunger rod (5, 25, 85) is arranged on the second base surface (4, 24, 84) of the cylindrical internal space (2, 22, 82) and supports the plunger rod (5, 25, 85) centrally in the region of the cylinder axis of the internal space (2, 22, 82).

7. The compressed gas motor (1, 21, 51, 81) according to one of the preceding claims,
**characterised in that**
the at least one passage (9, 29, 89) comprises a continuous, gas-permeable bore hole (9, 29) in the first plunger part (6, 26, 86), and in particular is such a bore (9, 29).

8. The compressed gas motor (1, 21, 51, 81) according to one of the preceding claims,
**characterised in that**
the distance between the first plunger part (6, 26, 86) and the second plunger part (7, 27, 87) is greater than 1 mm, preferably between 1 mm and 100 mm, particularly preferably between 5 mm and 10 mm.

9. The compressed gas motor (1, 21, 51, 81) according to one of the preceding claims,
**characterised in that**
the distance of the first opening (11, 31, 61, 91) from the closed first base surface (3, 23, 83) is at least equal to the height of the first plunger part (6, 26, 86) in the direction of the cylinder axis of the internal space (2, 22, 82).

10. The compressed gas motor (1, 21, 51, 81) according to one of the preceding claims,
**characterised in that**
the first plunger part (6, 26, 86) and/or the second plunger part (7, 27, 87) are cylindrical, at least in regions, and contact the internal wall of the cylindrical internal space (2, 22, 82) tightly.

11. The compressed gas motor (1, 21, 51, 81) according to one of the preceding claims,
**characterised in that**
a reservoir is connected at the first opening (11, 31, 61, 91) and contains a compressed gas and/or the second opening (12, 32, 62, 92) is connected to the surrounding environment of the compressed gas motor (1, 21, 51, 81) in a gas-permeable manner, preferably without any conduits (64) impeding the gas flow.

12. The compressed gas motor (1, 21, 51, 81) according to one of the preceding claims,
**characterised in that**
an attaching means (71, 72) for attachment of a lavage attachment, in particular a bayonet closure (72), are arranged externally in the region of the second base surface (4, 24, 84).

13. The compressed gas motor (1, 21, 51, 81) according to one of the preceding claims,
**characterised in that**
the end of the plunger rod (5, 25, 85) protruding from the second base surface (4, 24, 84) comprises a pestle (14, 34, 70), in particular a mushroom-shaped pestle (14, 34, 70).

14. The compressed gas motor (1, 21, 51, 81) according to Claim 13, **characterised in that**
the plunger parts (6, 7, 26, 27, 38, 86, 87), the connections (8, 28, 28', 88) of the plunger parts (6, 7, 26, 27, 86, 87), the plunger rod (5, 25, 85), the walls of the cylindrical internal space (2, 22, 82), the end(s) of the base surfaces (3, 4, 23, 24, 83, 84), the bracket, the restoring arrangement (10, 90), the attaching means (71, 72) and/or the pestle (14, 34, 70) are made of plastic material, in particular are injection moulded components.

15. The compressed gas motor (1, 21, 51, 81) according to one of the preceding claims,
**characterised in that**
the internal space (2, 22, 82) is the inside of a cylindrical tube and the compressed gas motor (1, 21, 51, 81) preferably substantially comprises, at least in regions, a cylindrical external surface.

16. The compressed gas motor (1, 21, 51, 81) according to one of the preceding claims,
**characterised in that**
the first opening (11, 31, 61, 91) can be closed completely by the first plunger part (6, 26, 86) and the second opening (12, 32, 62, 92) can be closed completely by the second plunger part (7, 27, 87) in alternating manner during an oscillating motion of the plunger in the internal space (2, 22, 82).

17. The compressed gas motor (1, 21, 51, 81) according to one of the receding claims,
**characterised in that**
the restoring arrangement (10, 90) comprises a spring, in particular a plastic spring, steel spring (10) and/or gas spring (90), arranged between the second plunger part (7, 27, 87) and the second base surface (4, 24, 84) of the cylindrical internal space (2, 22, 82).

18. The compressed gas motor (1, 21, 51, 81) according to one of Claims 1 to 16,
**characterised in that**
the restoring arrangement comprises a third plunger part (38),
a third opening (32') for discharge of a gas from the inside of the internal space (22), and
a fourth opening (31') for supplying a compressed gas into the internal space (22), wherein the third and fourth openings (32', 31') are arranged in the cylinder jacket of the cylindrical internal space (22),
the fourth opening (31') is arranged between the second opening (32) and the second base surface (24) of the cylindrical internal space (22),
the third opening (32') is arranged between the second opening (32) and the fourth opening (31'),
the third opening (32') can be closed by the second plunger part (27) and the fourth opening (31') can be closed by the third plunger part (38),
and wherein the third plunger part (38) is a part of the plunger that is arranged on the plunger rod (25) between the second plunger part (27) and the second base surface (24) of the cylindrical internal space (22) and is connected to the second plunger part (27), and
the third plunger part (38) comprises at least one passage (39) and/or forms at least one passage between the third plunger part (38) and the cylinder wall of the internal space (22).

19. The compressed gas motor (1, 21, 51, 81) according to Claim 18, **characterised in that**
the third plunger part (38) is structured similarly to the first plunger part (2, 26, 86) according to one of the preceding claims and/or the internal wall of the internal space (2, 22, 82) is structured with respect to the third plunger part (38) similarly to the manner in which it is structured with respect to the first plunger part (2, 26, 86) according to one of Claims 2 or 3.

20. The compressed gas motor (1, 21, 51, 81) according to Claim 18 or 19, **characterised in that**
the plunger rod (5, 25, 85) is connected rigidly and immovably to the plunger, and is preferably connected to the second plunger part (7, 27, 87) or the third plunger part (38), in particular in the region of the cylinder axis.

21. A handle (50), comprising a compressed gas motor (1, 21, 51, 81) according to one of the preceding claims and a valve (65) and an operating device (66) arranged at the handle (50) for controlling the valve (65), wherein the valve (65) is arranged in a conduit (63, 64), in particular a compressed gas conduit (63), that is connected to the first opening (11, 31, 61, 91) and/or second opening (12, 32, 62, 92), preferably to the first opening (11, 31, 61, 91), and particularly preferably to the first opening (11, 31, 61, 91) and fourth opening (31'), wherein the handle is intended for a lavage system.

22. A lavage system comprising the compressed gas motor (1, 21, 51, 81) according to one of Claims 13 to 20, preferably comprising a handle (50) according to Claim 21, further comprising a rinsing liquid reservoir and/or a connector for a rinsing liquid reservoir, wherein impacts of force can be transmitted by means of the end of the plunger rod (5, 25, 85) that protrudes beyond the second base surface (4, 24, 84), in particular by means of the pestle (14, 34, 70), to a force transmission apparatus, preferably a membrane, in order to generate spray puffs containing the rinsing liquid.

23. A method, for operating a compressed gas motor according to one of Claims 1 to 20,
**characterised by**
supplying a compressed gas through the first opening into an intermediate space between the first and second plunger part,
building up a positive pressure in the intermediate space,
moving the plunger parts in the internal space of the compressed gas motor due to the positive pressure,
closing the first opening and opening the second opening due to the movement of the plunger parts,
releasing the expanded gas through the second opening from the intermediate space, restoring the plunger parts in the internal space through a restoring arrangement.

## Revendications

1. Moteur à gaz comprimé (1, 21, 51, 81) pour le fonctionnement d'un système de lavage, comprenant un espace intérieur cylindrique (2, 22, 82) fermé de manière étanche au gaz au niveau d'au moins une première surface de base (3,23, 83),
au moins un piston agencé de façon mobile le long de l'axe de cylindre dans l'espace intérieur cylindrique (2, 22, 82) et scellant de façon étanche l'espace intérieur (2, 22, 82),
une tige de piston (5, 25, 85) reliée au piston et faisant saillie à partir de l'espace intérieur (2, 22, 82), au-delà d'une deuxième surface de base (4, 24, 84),
une première ouverture (11, 31, 61, 91) pour l'approvisionnement d'un gaz comprimé dans l'espace intérieur (2, 22, 82) et
une deuxième ouverture (12, 32, 62, 92) pour l'évacuation d'un gaz hors de l'espace intérieur (2, 22, 82),
**caractérisé en ce que**
les ouvertures (11, 12, 31, 32, 61, 62, 91, 92) sont agencées dans l'enveloppe cylindrique de l'espace intérieur cylindrique (2, 22, 82),
la première ouverture (11, 31, 61, 91) est agencée entre la deuxième ouverture (12, 32, 62, 92) et la première surface de base (3,23, 83) fermée,
le piston comprend une première partie de piston (6, 26, 86) et une deuxième partie de piston (7, 27, 87) reliées l'une à l'autre,
dans lequel la première partie de piston (6, 26, 86) comprend au moins un passage (9, 29, 89) et/ou forme au moins un passage entre la première partie de piston (6, 26, 86) et la paroi cylindrique de l'espace intérieur (2, 22, 82), et
la deuxième partie de piston (7, 27, 87) ferme de façon étanche l'intérieur de l'espace intérieur (2, 22, 82),
dans lequel au moins un passage (9, 29, 89) de la première partie de piston (6, 26, 86) relie un espace intérieur (2, 22, 82) entre la première et la deuxième partie de piston (7, 27, 87) à une région d'espace intérieur (16, 36, 96) entre la première surface de base (3,23, 83) fermée et la première partie de piston (6, 26, 86), et
dans lequel la première ouverture (11, 31, 61, 91) est refermable par la première partie de piston (6, 26, 86) et la deuxième ouverture (12, 32, 62, 92) est refermable par la deuxième partie de piston (7, 27, 87), et
le moteur à gaz comprimé (1, 21, 51, 81) comprend un dispositif de rappel (10, 90) exerçant au moins par moments une force sur le piston en direction de la première surface de base (3,23, 83) de l'espace intérieur cylindrique (2, 22, 82), lorsque la deuxième partie de piston (7, 27, 87) ne ferme pas complètement la deuxième ouverture (12, 32, 62, 92).

2. Moteur à gaz comprimé (1, 21, 51, 81) selon la revendication 1, **caractérisé en ce que**
la paroi de l'espace intérieur (2, 22, 82) comprend un sillon s'étendant de la région de fin de la première surface de base (3,23, 83) de l'espace intérieur (2, 22, 82) jusqu'à la région de la hauteur de la deuxième ouverture (12, 32, 62, 92) et formant au moins un passage entre la première partie de piston (6, 26, 86) et la paroi cylindrique de l'espace intérieur (2, 22, 82), le sillon n'étant pas relié aux ouvertures (11, 12, 31, 32, 61, 62, 91, 92).

3. Moteur à gaz comprimé (1, 21, 51, 81) selon la revendication 2, **caractérisé en ce que**
le sillon s'étend jusque dans la fin de la première surface de base (3,23, 83) de l'espace intérieur cylindrique (2, 22, 82), la première partie de piston (6, 26, 86) étant de préférence un corps cylindrique ne comprenant de façon particulièrement préférée pas d'autres passages (9, 29, 89).

4. Moteur à gaz comprimé (1, 21, 51, 81) selon l'une des revendications précédentes, **caractérisé en ce que**
le première partie de piston (6, 26, 86) et la deuxième partie de piston (7, 27, 87) sont reliées l'une à l'autre par un moyen d'assemblage rigide (8, 28, 28', 88), de préférence par le biais d'une barre (8, 28, 28'), de façon particulièrement préférée par un prolongement de la tige de piston (5, 25, 85), en particulier le long de l'axe de cylindre.

5. Moteur à gaz comprimé (1, 21, 51, 81) selon l'une des revendications précédentes, **caractérisé en ce que**
la deuxième surface de base (4, 24, 84) de l'espace intérieur cylindrique (2, 22, 82) est ouverte.

6. Moteur à gaz comprimé (1, 21, 51, 81) selon l'une des revendications précédentes, **caractérisé en ce que**
un support pour la tige de piston (5, 25, 85) supportant la tige de piston (5, 25, 85) de préférence au centre de la région de l'axe de cylindre de l'espace intérieur (2, 22, 82) est agencé sur la deuxième surface de base (4, 24, 84) de l'espace intérieur cylindrique (2, 22, 82).

7. Moteur à gaz comprimé (1, 21, 51, 81) selon l'une des revendications précédentes, **caractérisé en ce que**
l'au moins un passage (9, 29, 89) comprend au moins un perçage continu perméable au gaz (9, 29) dans la première partie de piston (6, 26, 86), en particulier **en ce qu'**il est un tel perçage (9, 29).

8. Moteur à gaz comprimé (1, 21, 51, 81) selon l'une des revendications précédentes, **caractérisé en ce que**
l'espacement entre la première partie de piston (6, 26, 86) et la deuxième partie de piston (7, 27, 87) est supérieur à 1 mm, de préférence compris entre 1 mm et 100 mm, de façon particulièrement préférée entre 5 mm et 10 mm.

9. Moteur à gaz comprimé (1, 21, 51, 81) selon l'une des revendications précédentes, **caractérisé en ce que**
l'espacement entre la première ouverture (11, 31, 61, 91) et la première surface de base (3,23, 83) germée est au moins aussi grand que la hauteur de la première partie de piston (6, 26, 86) dans la direction de l'axe de cylindre de l'espace intérieur (2, 22, 82).

10. Moteur à gaz comprimé (1, 21, 51, 81) selon l'une des revendications précédentes, **caractérisé en ce que**
la première partie de piston (6, 26, 86) et/ou la deuxième partie de piston (7, 27, 87) sont au moins par endroits cylindriques et s'appliquent de façon étanche contre la paroi intérieure de l'espace intérieur (2, 22, 82) cylindrique.

11. Moteur à gaz comprimé (1, 21, 51, 81) selon l'une des revendications précédentes, **caractérisé en ce que**
un réservoir contenant un gaz comprimé est raccordé à la première ouverture (11, 31, 61, 91) et/ou **en ce que** la deuxième ouverture (12, 32, 62, 92) est reliée de façon perméable au gaz à l'environnement du moteur à gaz comprimé (1, 21, 51, 81), de préférence sans conduites (64) susceptibles de gêner le flux de gaz.

12. Moteur à gaz comprimé (1, 21, 51, 81) selon l'une des revendications précédentes, **caractérisé en ce que**
un moyen de fixation (71, 72) destiné à fixer un raccord de lavage, en particulier une fermeture à baïonnette (72), est agencé à l'extérieur dans la région de la deuxième surface de base (4, 24, 84).

13. Moteur à gaz comprimé (1, 21, 51, 81) selon l'une des revendications précédentes, **caractérisé en ce que**
l'extrémité de la tige de piston (5, 25, 85) faisant saillie à partir de la deuxième surface de base (4, 24, 84) comprend un poussoir (14, 34, 70), en particulier un poussoir en champignon (14, 34, 70).

14. Moteur à gaz comprimé (1, 21, 51, 81) selon la revendication 13, **caractérisé en ce que**
les parties de piston (6, 7, 26, 27, 86, 87), les assemblages (8, 28, 28', 88) des parties de piston (6, 7, 26, 27, 86, 87), la tige de piston (5, 25, 85), les parois de l'espace intérieur cylindrique (2, 22, 82), la ou les parties finales des surfaces de base (3, 4, 23, 24, 83, 84), le support, le dispositif de rappel (10, 90), les moyens de fixation et/ou le poussoir (14, 34, 70) sont constitués de plastique, et sont en particulier des pièces moulées par injection.

15. Moteur à gaz comprimé (1, 21, 51, 81) selon l'une des revendications précédentes, **caractérisé en ce que**
l'espace intérieur (2, 22, 82) correspond à l'intérieur d'un tuyau cylindrique et **en ce que** le moteur à gaz comprimé (1, 21, 51, 81) comprend au moins par endroits, une surface extérieure cylindrique, de préférence quasiment cylindrique.

16. Moteur à gaz comprimé (1, 21, 51, 81) selon l'une des revendications précédentes, **caractérisé en ce que**
lors d'un mouvement d'oscillation du piston dans l'espace intérieur (2, 22, 82), la première ouverture (11, 31, 61, 91) peut être complètement fermée par la première partie de piston (6, 26, 86) et la deuxième ouverture (12, 32, 62, 92) par la deuxième partie de piston (7, 27, 87), de façon alternée.

17. Moteur à gaz comprimé (1, 21, 51, 81) selon l'une des revendications précédentes, **caractérisé en ce que**
le dispositif de rappel (10, 90) comprend un ressort, en particulier un ressort en plastique et acier (10) et/ou un ressort à gaz (90) agencés entre la deuxième partie de piston (7, 27, 87) et la deuxième surface de base (4, 24, 84) de l'espace intérieur cylindrique (2, 22, 82)

18. Moteur à gaz comprimé (1, 21, 51, 81) selon l'une des revendications 1 à 16,
**caractérisé en ce que**
le dispositif de rappel comprend une troisième partie de piston (38),
une troisième ouverture (32') pour l'évacuation d'un gaz hors de l'intérieur de l'espace intérieur (22), et
une quatrième ouverture (31') pour l'alimentation d'un gaz comprimé dans l'espace intérieur (22),
dans lequel les troisième et quatrième ouvertures (32', 31') sont agencées dans l'enveloppe cylindrique de l'espace intérieur cylindrique (22),
la quatrième ouverture (31') est agencée entre la deuxième ouverture (32) et la deuxième surface de base (24) de l'espace intérieur cylindrique (22),
la troisième ouverture (32') est agencée entre la deuxième ouverture (32) et la quatrième ouverture (31'),
la troisième ouverture (32') peut être fermée par la deuxième partie de piston (27) et la quatrième ouverture (31') peut être fermée par la troisième partie de piston (38),
et dans lequel la troisième partie de piston (38) est une partie du piston agencée sur la tige de piston (25) entre la deuxième partie de piston (27) et la deuxième surface de base (24) de l'espace intérieur cylindrique (22) et reliée à la deuxième partie de piston (27), et
la troisième partie de piston (38) comprend au moins un passage (39) et/ou forme au moins un passage entre la troisième partie de piston (38) et la paroi cylindrique de l'espace intérieur (22).

19. Moteur à gaz comprimé (1, 21, 51, 81) selon la revendication 18, **caractérisé en ce que**
la troisième partie de piston (38) est construite de la même façon que la première partie de piston (2, 26, 86) selon l'une des revendications précédentes, et/ou la paroi intérieure de l'espace intérieur (2, 22, 82) est construite de la même façon quant à la troisième partie de piston (38) que quant à la première partie de piston (6, 26, 86) selon l'une des revendications 2 ou 3.

20. Moteur à gaz comprimé (1, 21, 51, 81) selon la revendication 18 ou 19,
**caractérisé en ce que**
la tige de piston (5, 25, 85) est reliée au piston de manière rigide et immobile, de préférence à la deuxième partie de piston (7, 27, 87) ou à la troisième partie de piston (38), en particulier dans la région de l'axe de cylindre.

21. Poignée (50) comprenant un moteur à gaz comprimé (1, 21, 51, 81) selon l'une des revendications précédentes, ainsi qu'une soupape (65) et un dispositif de commande (66) agencé sur la poignée (50) pour commander la soupape (65), dans laquelle la soupape (65) est agencée dans une conduite (63, 64), en particulier une conduite de gaz comprimé (63), laquelle est reliée à la première ouverture (11, 31, 61, 91) et/ou à la deuxième ouverture (12, 32, 62, 92), de préférence à la première (11, 31, 61, 91) et de façon particulièrement préférée à la première (11, 31, 61, 91) et à la quatrième ouverture (31'), la poignée étant prévue pour un système de lavage.

22. Système de lavage comprenant un moteur à gaz comprimé (1, 21, 51, 81) selon l'une des revendications 13 à 20, comprenant de préférence une poignée (50) selon la revendication 21, comprenant en outre un réservoir de liquide de rinçage et/ou un raccord pour un réservoir de liquide de rinçage, dans lequel des coups de force peuvent être transmis à un dispositif de transmission de force, de préférence une membrane, par le biais de l'extrémité de la tige de piston (5, 25, 85) faisant saillie hors de la deuxième surface de base (4, 24, 84), en particulier par le biais d'un poussoir (14, 34, 70), afin de produire des coups de pulvérisation avec le liquide de rinçage.

23. Procédé pour le fonctionnement d'un moteur à gaz comprimé selon l'une des revendications 1 à 20, **caractérisé par**
- l'alimentation d'un gaz comprimé à travers la première ouverture dans un espace intermédiaire entre les première et deuxième parties de piston,
- la génération d'une surpression dans l'espace intermédiaire),
- le déplacement des parties de piston dans l'espace intérieur du moteur à gaz comprimé en raison de la surpression,
- l'évacuation du gaz expansé hors de l'espace intermédiaire par la deuxième ouverture,
- le rappel des parties de piston dans l'espace intérieur par un dispositif de rappel.
